# EUROPEAN PATENT SPECIFICATION

(11) **EP 0 614 699 B1**
(45) Date of publication and mention of the grant of the patent: **04.06.1997**
(21) Application number: 94103027.2
(22) Date of filing: 01.03.1994
(51) Int. Cl.: B01J 38/60, B01J 27/30, C07C 2/62

(54) **Process for the recovery of spent fluorinated sulphonic acid catalyst in an alkylation process**
Verfahren zur Rückgewinnung verbrauchter fluorierter Sulfansäure-Katalysatoren für ein Alkylierungsverfahren
Procédé de récupération d'un catalyseur usé à base d'acide sulferique fluoré utilisé dans un procédé d'alkylation

(30) Priority: 12.03.1993 DK 287/93
(43) Date of publication of application: 14.09.1994
(73) Proprietor: Haldor Topsoe A/S, DK-2800 Lyngby (DK)
(72) Inventor: Hommeltoft, Sven Ivar, DK-3400 Hillerod (DK)
(74) Representative: Grünecker, Kinkeldey, Stockmair & Schwanhäusser Anwaltssozietät

(56) References cited:
- EP-A- 0 433 954
- GB-A- 2 139 624
- US-A- 4 096 199
- US-A- 5 100 850

## Description

The present invention is directed to improvements in catalyzed alkylation of aliphatic hydrocarbons.

More particulary, the invention is related to the recovery of a fluorinated sulphonic acid catalyst from acid tar being formed as by-product during alkylation of hydrocarbons.

In US Patent No. 4,096,199 a method for the recovery of fluorosulphuric acid catalyst is suggested by which the acid is regenerated from an acid-water mixture by addition of sulphur trioxide to a gase phase resulting from the acid-water mixture and containing hydrogen fluoride. By reaction of sulphur trioxide the fluorosulphuric acid catalyst is regenerated from hydrogen fluoride.

Use of proton donating acid for recovering acetic acid is disclosed in GB Patent Application No. 2,139,624.

Acid catalyzed alkylation of aliphatic hydrocarbons with olefinic hydrocarbons is a well known process for the preparation of high octane gasoline products. Alkylation of aliphatic hydrocarbons is generally accomplished in the liquid phase by reacting paraffins and olefins in the presence of a strong acid catalyst.

Utilization of fluorinated sulphonic acids as efficient alkylation catalysts in the alkylation of aliphatic hydrocarbons with olefins is disclosed in European Patent Application No. 433,954. By the disclosed process, a process stream including a hydrocarbon substrate and an olefinic alkylating agent is reacted in contact with a fluorinated sulphonic acid catalyst in a fixed bed alkylation reactor containing polar contact material. On the contact material is established a reaction zone with the fluorinated sulphonic acid catalyst adsorbed within a confined area of the contact material. In the reaction zone, the process stream is converted at alkylating conditions to a product stream of alkylated hydrocarbons by catalysis of the fluorinated sulphonic acid adsorbed on the contact material.

During the alkylation reaction, the acid catalyst and, consequently, the reaction zone, moves as a well-defined band between the ends of the reactor due to interaction with the process stream flowing through and reacting in the zone.

Although it is possible to reuse the acid catalyst as it reaches the outlet end of the alkylation reactor by reversing the flow direction of the process stream introduced into the alkylation reactor, small amounts of the acid catalyst will continuously be trapped in tar by-product being formed by side reactions during the process. The tar adsorbs like the acid catalyst as a movable band on the support material adjacent to the reaction zone. It is, thus, possible to withdraw the tar from the reactor, whenever the tar band reaches one of the ends of the reactor.

Even if the tar contains only small amounts of spent acid catalyst, it is desirable to recover the catalyst from the tar in order to improve the economy of the alkylation process. Conventionally methods, like distillation or extraction of the acid directly from the tar, are inefficient because of strong interactions between the sulphonic acid and basic components in the tar.

It is, therefore, a principal object of this invention to provide a process for the efficient recovery of fluorinated sulphonic acid catalyst from an alkylation process.

Accordingly, a broad embodiment of the invention is directed towards a process for the recovery of fluorinated sulphonic acid catalyst from tar being formed during alkylation of hydrocarbons in the presence of the fluorinated sulphonic acid catalyst and containing spent fluorinated sulphonic acid catalyst in form of salts with basic components in the tar, which process comprises,
treating the tar with a proton donating acid by mixing of the acid and the tar, thereby, converting the spent catalyst to the fluorinated sulphonic acid;
separating the fluorinated sulphonic acid from the tar-acid mixture by stripping the acid in gaseous form from the mixture with an inert stripping agent at a temperature of 0-200°C and pressure of 0.00013-0.0004 bar or 0.0007-1 bar (atm); and finally
recovering the separated fluorinated sulphonic acid from the stripping agent.

Without being limited by the following theory, it is believed, as previously indicated, that the fluorinated sulphonic acid is in the tar strongly bound to basic components, consisting mainly of dienes groups, such as cyclopentadienes, which are formed by side reactions during alkylation of aliphatic hydrocarbons.

When treating the tar with a proton donating acid, preferably sulphuric acid or phosphoric acid, the acid competes with the fluorinated sulphonic acid for the basic components of the tar. Amounts of the proton donating acid added to the tar will be typically in range of 1-100 mole equivalents proton donating acid per mole fluorinated sulphonic acid in the tar. Fluorinated sulphonic acids are very strong acids, and an equilibrium between free fluorinated sulphonic acid and salts of the acid with the basic components in the tar will be established.

The equilibrium may be changed in favour of formation of the free fluorinated sulphonic acid when removing the free acid continuously from the tar-acid mixture, as in the inventive process, by stripping the mixture with an inert stripping agent, and, thereby, continuously removing liberated fluorinated sulphonic acid from the mixture.

Suitable stripping agents are selected from compounds which are inert with respect to components and acids being present in the mixture and having a boiling point below that of sulphuric acid at the desired stripping temperature and pressure used in the process. Presently, the most preferred stripping agents are selected from the group of C₁₀-C₁₄ aliphatic hydrocarbons.

Presence of water in the tar results in more unfavourable recovery efficiency for the fluorinated sulphonic acid, caused by the basicity of the water. In order to obtain satisfying recovery rates of the sulphonic acid, water must be removed from the tar. This is in the inventive process obtained by addition of oleum, i.e. fuming sulphuric acid containing preferably 2-35 wt% SO₃, which in situ removes water by reaction of SO₃ with water to sulphuric acid.

During operation of the process in large scale, stripping of the tar-acid mixture will be carried out in a stripping column equipped with valve trays or filler bodies. The tar-acid mixture is continuously introduced at the top of the column and is withdrawn at the bottom. The stripping agent is introduced at the bottom of the column and flows in gaseous form upwardly and countercurrently with the tar-acid mixture towards the top of the column. When passing through the column, the stripping agent comes into intimate contact with the mixture and the free fluorinated sulphonic acid, being in the gaseous phase at the stripping conditions used in the column, is stripped from the tar-acid mixture by vapours of the stripping agent. Stripped fluorinated sulphonic acid is withdrawn at the top of the column together with vapours of the stripping agent. The fluorinated sulphonic acid is finally separated from the stripping agent by conventionel separation techniques, comprising phase separation, condensation, distillation, adsorption and the like.

### Example 1

25 ml tar from the alkylation of isobutane with trifluoromethansulphonic acid (CF₃SO₃H) as catalyst, having a density of 1.20 g/ml and a content of 53.6 g CF₃SO₃H/g tar and 2.02 g H₂O/g, were mixed with 25 ml 100% sulphuric acid in a 100 ml flask. To the obtained tar-sulphuric acid mixture 25 ml dodecane were added. The mixture was then stripped by heating in a water bath and evacuating while stirring. A first fraction was stripped off of the mixture at 30-60°C and a pressure of 0.00013-0.0004 bar (0.1-0.3 mm Hg), which fraction consisted of two phases: a heavy acid phase and a lighter dodecane phase. A second fraction was stripped off at a bath temperature of about 70°C, and a pressure of 0.0007-0.0011 bar (0.05-0.8 mm Hg). The second fraction consisted of two phases. Both the first and second fraction were extracted with water. The CF₃SO₃H-content in the aqueous extract of the fractions was analyzed and determined by ion chromatography analysis. The first fraction contained 6.12 g and the second fraction 7.90 g of CF₃SO₃H, resulting in a total yield of 14.02 g acid or 87% recovery. The remaining amount of CF₃SO₃H in the tar was found in the remanence from the distillation.

### Example 2

50 ml of acid tar were mixed with 60 ml 10% oleum and 50 ml dodecane added. The mixture was stripped as described in Example 1. The total yield of CF₃SO₃H recovered was 29.5 g corresponding to 92% recovery. The remaining 8% of the acid were found in the remanence.

## Claims

1. Process for the recovery of fluorinated sulphonic acid catalyst from tar being formed during alkylation of hydrocarbons in the presence of the fluorinated sulphonic acid catalyst and containing spent fluorinated sulphonic acid catalyst in form of salts with basic components in the tar, which process comprises,
treating the tar with a proton donating acid by mixing of the acid and the tar, thereby, converting the spent catalyst to the fluorinated sulphonic acid;
separating the obtained fluorinated sulphonic acid from the tar-acid mixture by stripping off the acid from the mixture with an inert stripping agent at a temperature of 0-200°C and pressure of 0.00013-0.0004 bar or 0.0007-1 bar (atm); and finally
recovering the separated fluorinated sulphonic acid from the stripping agent.

2. The process of claim 1, wherein the proton donating acid for treatment of the tar comprises sulphuric acid and/or phosphoric acid.

3. The process of claim 1, wherein the proton donating acid for treatment of the tar is oleum.

4. The process of claim 1, wherein the stripping agent is selected from the group of inert compounds having a boiling point below the boiling point of the proton donating acid at the temperature and pressure being used during the separation step.

5. The process of claim 4, wherein the stripping agent is selected from the group of C₁₀-C₁₄ aliphatic hydrocarbons.

6. The process of claim 1, wherein the fluorinated sulphonic acid catalyst consists of trifluoromethanesulphonic acid.

7. The process of any one of the preceding claims, wherein the separation of the spent fluorinated sulphonic acid catalyst from the tar-acid mixture is carried out continuously in a stripping column with the mixture passing in countercurrent flow with the stripping agent through the column.

## Patentansprüche

1. Verfahren zur Rückgewinnung eines fluorierten Sulfonsäure-Katalysators aus Teer, welcher während der Alkylierung von Kohlenwasserstoffen in Gegenwart des fluorierten Sulfonsäure-Katalysators gebildet wird und verbrauchten fluorierten Sulfonsäure-Katalysator in Form von Salzen mit basischen Bestandteilen in dem Teer enthält, wobei dieses Verfahren umfaßt:
Behandeln des Teers mit einer protonenabgebenden Säure durch Vermischen der Säure und des Teers, wodurch der verbrauchte Katalysator in die fluorierte Sulfonsäure umgewandelt wird;
Abtrennen der erhaltenen fluorierten Sulfonsäure von dem Teer-Säure-Gemisch durch Abstrippen der Säure aus dem Gemisch mit einem inerten Abtreibmittel bei einer Temperatur von 0 bis 200°C und einem Druck von 0,00013 bis 0,0004 bar oder 0,0007 bis 1 bar (atm); und schließlich
Rückgewinnen der abgetrennten fluorierten Sulfonsäure von dem Abtreibmittel.

2. Verfahren nach Anspruch 1, worin die protonenabgebende Säure Zur Behandlung des Teers Schwefelsäure und/oder Phosphorsäure umfaßt.

3. Verfahren nach Anspruch 1, worin die protonenabgebende Säure zur Behandlung des Teers Oleum ist.

4. Verfahren nach Anspruch 1, worin das Abtreibmittel ausgewählt ist aus der Gruppe von inerten Verbindungen mit einem Siedepunkt unter dem Siedepunkt der protonenabgebenden Säure bei der Temperatur und dem Druck, die während des Abtrennschritts verwendet werden.

5. Verfahren nach Anspruch 4, worin das Abtreibmittel aus der Gruppe von aliphatischen C₁₀-C₁₄-Kohlenwasserstoffen ausgewählt ist.

6. Verfahren nach Anspruch 1, worin der fluorierte Sulfonsäure-Katalysator aus Trifluormethansulfonsäure besteht.

7. Verfahren nach einem der vorhergehenden Ansprüche, worin die Abtrennung des verbrauchten fluorierten Sulfonsäure-Katalysators von dem Teer-Säure-Gemisch kontinuierlich in einer Abtreibkolonne durchgeführt wird, wobei das Gemisch die Kolonne im Gegenstrom zu dem Abtreibmittel durchläuft.

## Revendications

1. Procédé pour la récupération d'un catalyseur à base d'acide sulfonique fluoré, à partir de goudron formé au cours de l'alkylation d'hydrocarbures en présence du catalyseur à base d'acide sulfonique fluoré et contenant du catalyseur à base d'acide sulfonique fluoré usé, sous forme de sels avec des constituants basiques du goudron, ce procédé comprenant :
le traitement du goudron par un acide donneur de protons, par mélange de l'acide et du goudron, en convertissant ainsi le catalyseur usé en de l'acide sulfonique fluoré,
la séparation de l'acide sulfonique fluoré ainsi obtenu à partir du mélange goudron-acide, par enlèvement par entraînement de l'acide du mélange à l'aide d'un agent inerte d'entraînement, à une température de 0 à 200°C et sous une pression de 0,00013 à 0,0004 bar ou de 0,0007 à 1 bar (atmosphère); et, enfin,
la récupération de l'acide sulfonique fluoré séparé de l'agent d'entraînement.

2. Procédé selon la revendication 1, dans lequel l'acide donneur de protons destiné au traitement du goudron comprend de l'acide sulfurique et/ou de l'acide phosphorique.

3. Procédé selon la revendication 1, dans lequel l'acide donneur de protons destiné au traitement du goudron est de l'oléum.

4. Procédé selon la revendication 1, dans lequel l'agent d'entraînement est choisi dans le groupe des composés inertes ayant un point d'ébullition inférieur au point d'ébullition de l'acide donneur de protons, à la température et sous la pression utilisée au cours de l'étape de séparation.

5. Procédé selon la revendication 4, dans lequel l'agent d'entraînement est choisi dans le groupe des ensembles hydrocarbures aliphatiques en C₁₀-C₁₄.

6. Procédé selon la revendication 1, dans lequel le catalyseur à base d'acide sulfonique fluoré consiste en de l'acide trifluoro méthane sulfonique.

7. Procédé selon l'une quelconque des revendications précédentes, dans lequel la séparation du catalyseur à base d'acide sulfonique fluoré usé à partir du mélange goudron-acide est effectué en continu dans une colonne d'entraînement, le mélange traversant la colonne en un écoulement à contre-courant de l'agent d'entraînement.
